# EUROPEAN PATENT APPLICATION

(11) **EP 4 822 008 A1**
(43) Date of publication of application: **14.10.2026**
(21) Application number: 24900648.7
(22) Date of filing: 04.12.2024
(51) Int. Cl.: C12M 1/00, B01J 20/10, B01J 20/28, B01J 20/30, C12N 15/10, G01N 30/00

(54) **NUCLEIC ACID-BINDING PARTICLE AND METHOD FOR PRODUCING NUCLEIC ACID-BINDING PARTICLE**

(30) Priority: 08.12.2023 JP 2023207662
(71) Applicant: NIKON CORPORATION, Tokyo 140-8601 (JP)
(72) Inventor: NOZAKI Kiyoshi, Tokyo 140-8601 (JP); TAKAHASHI Youhei, Tokyo 140-8601 (JP); KUNINOBU HEYA Arisa, Tokyo 140-8601 (JP); TAKAHASHI Yuki, Tokyo 140-8601 (JP); MOROZUMI Akihiko, Tokyo 140-8601 (JP); YAMAO Ryoko, Tokyo 140-8601 (JP); TAKATSUKA Kenji, Tokyo 140-8601 (JP)
(74) Representative: Hoffmann Eitle
(86) International application number: PCT/JP2024/042893
(87) International publication number: WO 2025/121354

(57) **Abstract**

Provided is nucleic acid-binding particle, having at least a portion of a surface covered with silica, wherein a sphericity determined from the following formula (A) is 0.86 or more, and the surface has vicinal silanol groups: 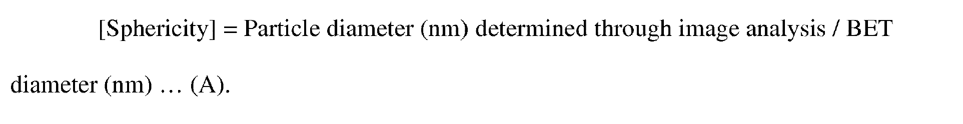 [Sphericity] = Particle diameter (nm) determined through image analysis/BET diameter (nm) (A).

## Description

### TECHNICAL FIELD

The present invention relates to a nucleic acid-binding particle and a method for producing a nucleic acid-binding particle.

This application claims priority based on Japanese Patent Application No. 2023-207662, filed on December 8, 2023, the contents of which are incorporated herein by reference.

### BACKGROUND ART

In recent years, improvements in the detection sensitivity of instruments, the widespread adoption of next-generation sequencers, and advancements in computer performance have led to the widespread use of methods for visualizing and quantifying gene and protein expression in individual cells present in biological tissues, and analyzing them along with their location information within the tissue.

Due to the above methods, there is a growing demand for efficiently recovering nucleic acids from nucleic acid-containing samples and performing genome analysis. For example, magnetic particles are known that efficiently obtain nucleic acids by adsorbing nucleic acids onto magnetic particles and recovering the nucleic acid-adsorbed particles using magnetic force (see, for example, Patent Document 1).

Furthermore, the nucleic acids recovered by the method described in Patent Document 1 may be nucleic acids from living organisms or synthetic nucleic acids. In other words, the "sample containing nucleic acids" may be a biological sample containing nucleic acids from living organisms or a sample containing synthetic nucleic acids.

In this specification, the term "biological sample" refers to a sample derived from a living organism that contains nucleic acids of that organism. The term "nucleic acids of a living organism" includes both DNA and RNA contained in the cells of that organism. Nucleic acids may be DNA and RNA contained in the cells themselves, or they may be biopolymers in which parts of DNA or RNA are deleted.

### Citation List

### Patent Document

Patent Document 1: Japanese Unexamined Patent Publication, First Publication No. 2023-046202

### SUMMARY OF INVENTION

### Technical Problem

The analytical methods described above have been attracting attention as a technology that can lead to the early detection of diseases by analyzing the genome contained in tissues in minute regions, such as early-stage cancers. From such a perspective, there is still room for improvement in particles for recovering nucleic acids, assuming high-efficiency nucleic acid recovery.

The present invention has been made in view of these circumstances, and aims to provide a nucleic acid-binding particle that enables efficient recovery of nucleic acids from a sample. It also aims to provide a method for easily producing such a nucleic acid-binding particle.

### Solution to Problem

One aspect of the present invention is a nucleic acid-binding particle, having at least a portion of a surface covered with silica, wherein
a sphericity determined from the following formula (A) is 0.86 or more, and the surface has vicinal silanol groups: 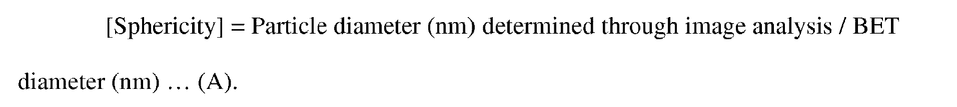 [Sphericity] = Particle diameter (nm) determined through image analysis/BET diameter (nm) (A).

One aspect of the present invention is a method for producing a nucleic acid-binding particle, comprising:
controlling a hydrophilicity evaluation value of a surface determined from the following formula (B) to 0.35 or more by subjecting a particle whose surface is covered with silica to a treatment for increasing vicinal silanol groups: $S = {α}_{H 2 O} / {α}_{N 2}$ (B)
S: Hydrophilicity evaluation value of surface of particle
α_{H2O}: BET specific surface area of binding particle determined through water vapor adsorption measurement
α_{N2}: BET specific surface area of binding particle determined through nitrogen adsorption measurement

### Advantageous Effects of Invention

According to the present invention, it is possible to provide a nucleic acid-binding particle capable of efficiently recovering nucleic acids from a biological sample. Furthermore, it is possible to provide a method for producing a nucleic acid-binding particle that enables easy production of such a nucleic acid-binding particle.

### BRIEF DESCRIPTION OF DRAWINGS

FIG. 1 is an explanatory diagram illustrating binding particle 100.
FIG. 2 is a flowchart illustrating a method for producing a nucleic acid-binding particle.
FIG. 3 is a flowchart showing a method for extracting nucleic acids using a nucleic acid-binding particle.
FIG. 4 is a graph showing evaluation results.
FIG. 5 is a graph showing evaluation results.
FIG. 6 is a graph showing evaluation results.

### DESCRIPTION OF EMBODIMENTS

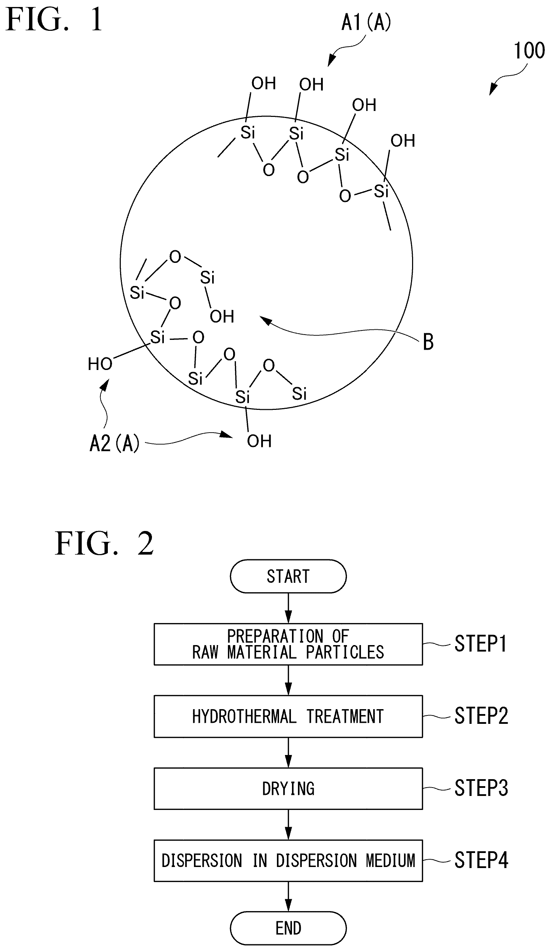

The nucleic acid-binding particle, the method for producing a nucleic acid-binding particle, and the nucleic acid extraction method according to the present embodiment will be described below with reference to FIGS. 1 to 3. Note that in all the following drawings, the dimensions and proportions of each component have been appropriately altered for clarity.

### <<Nucleic acid-binding particle>>

The nucleic acid-binding particle of the present embodiment is a particle in which at least a part of its surface is covered with silica, and has vicinal silanol groups on the surface. In the following description, the nucleic acid-binding particle may be simply referred to as a "binding particle".

The binding particle is used in the "Boom method", which is a technique for isolating nucleic acids from a biological sample. The Boom method isolates nucleic acids by utilizing the fact that nucleic acids are solid-phase adsorbed onto silica beads (binding particle) in the presence of a chaotropic substance. Specifically, the nucleic acids are adsorbed onto the silica beads in a biological sample containing nucleic acids and contaminants, and then the nucleic acids are separated from the biological sample together with the beads.

It should be noted that the Boom method in which the binding particle of the present embodiment is used refers to the Boom method in a broad sense, which includes the operation of adsorbing nucleic acids onto silica beads and recovering the nucleic acids, and is not limited to the original (narrow sense) Boom method. For example, it may be a modified version of the original Boom method in which changes have been made to the method of washing the silica beads or the method of separating the nucleic acids bound to the silica beads.

As products applying this principle, many nucleic acid-binding particles are commercially available. Among them, as described in the above-mentioned Patent Document 1, there have been proposed, manufactured, and sold nucleic acid-binding particles in which the behavior of beads can be controlled by magnetic force by manufacturing the particles with a magnetic material.

The inventors focused on the surface of the binding particle in order to recover nucleic acids more efficiently than with conventional nucleic acid-binding particles. After diligent research, the inventors focused on silanol groups exposed on the particle surface, which are considered to contribute to nucleic acid adsorption, and found that a binding particle with vicinal silanol groups on its surface exhibits high effectiveness, thus completing the invention.

As will be described in more detail later, the nucleic acid-binding particle of the present embodiment has an appropriate surface shape that facilitates the formation of vicinal silanol groups. As a result, the nucleic acid-binding particle of the present embodiment can easily adsorb (capture) nucleic acids with the vicinal silanol groups on the particle surface, making it possible to recover nucleic acids effectively.

### [Vicinal silanol groups]

FIG. 1 is an explanatory diagram illustrating binding particle 100. Binding particle 100 has a surface covered with silica. In FIG. 1, it is shown that the entire binding particle 100 is formed of silica.

FIG. 1 illustrates the silanol groups of binding particle 100. As shown in FIG. 1, the silica covering the surface of binding particle 100 has silanol groups. These silanol groups can be divided into surface silanol groups (silanol group A) present on the surface of the particle and internal silanol groups (silanol group B) present inside the particle. Furthermore, the surface silanol groups can be divided into vicinal silanol groups A1 and isolated silanol groups A2.

Here, the term "vicinal silanol groups" refers to hydroxyl groups present on the silica covering the surface of a particle (particle surface) and bonded to two adjacent silicon atoms via the oxygen in a siloxane bond (Si-O-Si).

Furthermore, the term "isolated silanol groups" refers to hydroxyl groups bonded to silicon atoms present on a particle surface, wherein no hydroxyl group exposed on the particle surface is present on an adjacent silicon atom bonded via oxygen to the silicon atom.

Binding particle 100 has at least a portion of its surface covered with silica. That is, at least a portion of the surface of binding particle 100 is composed of silica, and silica is exposed on the surface. The silica exposed on the surface of binding particle 100 has vicinal silanol groups A1. The vicinal silanol groups A1 are silanol groups that are directly bonded to the silica constituting the surface of the binding particle. In this sense, a configuration in which the surface of a silica particle is modified with an organic group and the organic group has a silanol group at its tip cannot be said to have vicinal silanol groups A1, and does not fall under the binding particle of the present embodiment.

Furthermore, whether a silanol group on the particle surface is a vicinal silanol group A1 or an isolated silanol group A2 can be confirmed by Raman spectroscopy. In the present embodiment, "having vicinal silanol groups on the surface" means "the peak of the vicinal silanol groups can be confirmed in the Raman spectrum".

### (Raman spectroscopy)

Raman spectroscopy can be performed using a known Raman spectrometer (for example, a Raman microscope in Via Reflex, manufactured by Renishaw).

When using the above Raman microscope, a 532 nm laser is applied as the excitation wavelength, and the measurement range is set from 690 cm⁻¹ to 4,000 cm⁻¹. The Raman shift is calibrated with reference to 520.5 cm⁻¹, derived from single-crystal Si. For the obtained spectrum, baseline correction and peak separation are carried out by data processing software WiRE 4.2 attached to the device. In addition, the peak intensity is normalized using the Raman peak intensity of approximately 800 cm⁻¹ derived from the Si-O framework.

In the resulting Raman spectrum, the peak for vicinal silanol groups appears at 3,610 cm⁻¹, and the peak for isolated silanol groups appears at 3,750 cm⁻¹.

### [Particle surface state 1: Sphericity]

As mentioned above, assuming a model in which nucleic acids bind to vicinal silanol groups on the surface of a binding particle, it is natural to assume that increasing the surface area is preferable to increasing the amount of nucleic acid that can be bound. Generally, to increase the surface area, particles with uneven surfaces or porous particles are considered preferable. However, contrary to this expectation, it was found that in the binding particle of the present embodiment, a smooth surface that approaches a perfect sphere is preferable. In other words, the binding particle is not porous and has a particle shape that is close to a perfect sphere.

The shape of the binding particle is evaluated using the sphericity determined from the following formula (A). The larger the sphericity, the closer the shape of the binding particle is to a perfect sphere. 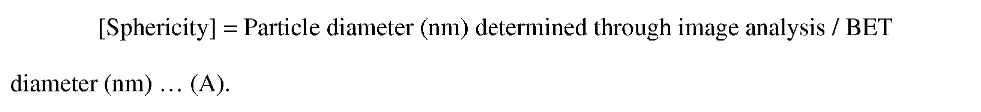 [Sphericity] = Particle diameter (nm) determined through image analysis/BET diameter (nm) (A).

The BET diameter of the binding particle can be determined from the following formula using α_{N2} (the BET specific surface area of the binding particle obtained by nitrogen adsorption measurement) and the density of the binding particle. $\left[BET diameter\right] = 6 / {α}_{N 2} \times \left(density of binding particle\right)$

The BET specific surface area is a value measured by the BET (Brunauer, Emmett, Teller) method, which is a gas adsorption method. The specific surface area measured by the gas adsorption method can be measured using known gas adsorption measuring devices (e.g., BELSORP mini II, BELSORP MAX II, both manufactured by Microtrac-Bell Co., Ltd.).

When measuring α_{N2}, nitrogen (for example, G1 grade nitrogen, manufactured by Taiyo Nippon Sanso Corporation) is used as the adsorbate.

Before measurement, the binding particle to be measured is subjected to vacuum degassing treatment at 100°C overnight using a known pretreatment device (e.g., Belprep vac II, manufactured by Microtrac-Bell Co., Ltd.) to remove any moisture physically adsorbed on the surface.

For the particle diameter determined through the image analysis of the binding particle, a value measured using a known measuring device for scanning electron microscopes is adopted. For example, an SEM image can be taken using a scanning electron microscope (manufactured by Hitachi High-Technologies Corporation, Scanning Electron Microscope S-3400N) at an acceleration voltage of 3 kV and magnification of 10,000x. Based on the obtained SEM image, the particle diameter can be automatically detected using software for calculating particle diameter (for example, MacView version 4, manufactured by Mountec Co., Ltd.).

Therefore, when the average particle diameters of binding particles are the same, the particle with a sphericity close to 1 is preferable.

The sphericity of the binding particle is preferably 0.86 or more, and more preferably 0.88 or more. The ideal upper limit for sphericity is "1".

The average particle diameter of the binding particle is preferably between 0.1 µm and 1,000 µm. Even if the average particle diameter of the binding particle exceeds 1,000 µm, the effect of the invention, which is to efficiently recover nucleic acids from biological samples, is still achieved. However, when using a binding particle with a larger particle diameter, it becomes necessary to increase the amount of reaction solution required for processing. Therefore, when the objective is to efficiently recover nucleic acids from "small-scale biological samples", the average particle diameter of the binding particle is preferably between 0.1 µm and 200 µm, more preferably between 0.1 µm and 50 µm, and even more preferably between 0.1 µm and 10 µm.

### [Particle surface state 2: Hydrophilicity of particle surface]

As described above, the binding particle of the present embodiment has a high surface hydrophilicity due to having vicinal silanol groups. The hydrophilicity of such a particle surface is evaluated by an evaluation value calculated according to the following formula (B). $S = {α}_{H 2 O} / {α}_{N 2}$ (B)
S: Hydrophilicity evaluation value of surface of particle
α_{H2O}: BET specific surface area (m²/g) of binding particle determined through water vapor adsorption measurement
α_{N2}: BET specific surface area (m²/g) of binding particle determined through nitrogen adsorption measurement.

α_{H2O} can be determined through the BET specific surface area measurement method described in [Particle surface state 1: Sphericity]. When measuring α_{H2O}, water vapor (for example, pure water that has been degassed three or more times) is used as the adsorbate. Pure water produced by a pure water production system (for example, Elix Advantage 5, manufactured by Merck KGaA) can be used.

It is more preferable that the evaluation value obtained by formula (B) above for the binding particle is 0.35 or more.

Furthermore, since the BET specific surface area is used in calculating the hydrophilicity evaluation value, it is considered to be strongly influenced by the sample amount (total surface area) of the binding particle used for measuring the specific surface area. As a result of studies by the inventors, it was found that even with a binding particle formed of the same material and differing only in particle diameter, that is, even with a binding particle having the same distribution state of vicinal silanol groups on the surface, the hydrophilicity evaluation value tends to increase as the particle diameter of the binding particle increases.

From formula (B) above, the theoretical upper limit of the evaluation value is "1". On the other hand, experimentally, it was found that a binding particle with an average particle diameter exceeding, for example, 10 µm, and with many vicinal silanol groups on its surface, had an evaluation value that greatly exceeded 1 as determined through formula (B) above.

For the binding particle with a large average particle diameter and many vicinal silanol groups, the result of the above evaluation value exceeding 1 was reproducible, and it was confirmed that nucleic acids can be suitably recovered even from the particle with an evaluation value exceeding 1 (see the Examples described below). Therefore, the inventors determined that a binding particle has a "high hydrophilicity" if the calculated evaluation value is 0.35 or more.

Based on the above findings and experimental confirmation results by the inventors, it is preferable that the binding particle of the present embodiment has an evaluation value of 0.35 or more and 130 or less calculated by formula (B) above.

### [Particle surface state 3: Abundance ratio of vicinal silanol groups]

Furthermore, it is preferable that the binding particle has an abundance ratio of vicinal silanol groups of 0.93 to 1.00, as determined from the following formula (C). $\left[Abundance ratio of vicinal silanol groups\right] = Rv / Rt$ (C)
(Rv: Peak area of vicinal silanol groups in Raman spectrum,
Rt: Total peak area of vicinal silanol groups and isolated silanol groups in Raman spectrum)

In formula (C), Rv and Rt can be determined from a Raman spectrum obtained by performing Raman spectroscopy on the binding particle based on the description in the above-mentioned section of (Raman spectroscopy).

The abundance ratio of vicinal silanol groups to the amount of silanol groups present on the surface is preferably high, and more preferably 0.93 or more. The upper limit of the abundance ratio of vicinal silanol groups is preferably " 1 ".

### [Particle structure]

The binding particle only needs to have at least a portion of its surface composed of silica. That is, the binding particle only needs to have silica exposed on at least a portion of its surface, and, for example, a portion of the particle surface may be coated with a resin. It is preferable that the binding particle is composed entirely of silica.

Furthermore, while at least a portion of the surface of the binding particle is composed of silica, the interior of the particle can be composed of various materials. The interior of the binding particle may be composed of materials other than silica. Examples of the "materials other than silica" include resin materials with a lower specific gravity than silica and metals with a higher specific gravity than silica.

It is preferable that the binding particle contains a magnetic material internally. The inclusion of a magnetic material in the binding particle allows for control of the particle's behavior using magnetic force, facilitating nucleic acid separation by the Boom method.

Such a binding particle can be manufactured, for example, by coating the surface of the particle with iron, and then further coating the surface of the iron-coated particle with silica.

As a method for coating the particle surfaces with iron, for example, the method described in "Engineered Magnetic Core-Shell SiO2/Fe Microspheres and "Medusa-like" Microspheres of SiO2/Iron Oxide/Carbon Nanofibers or Nanotubes" Langmuir 2014, 30, 32, 9850-9858 can be adopted.

Furthermore, as a method for further coating the surface of the iron-coated particle with silica, the method described in J. Am. Chem. Soc. 2008, 130, 28-29 can be adopted.

The binding particle may also be a silica particle composed of silica even internally.

Silica particles are commercially available as a gap material for electrical and electronic devices such as displays, and as a filler for semiconductor encapsulants. The inventors examined the vicinal silanol groups on the particle surfaces of these electronic materials, which belong to a completely different technological field from that of the nucleic acid-binding particles, and found that some materials can be repurposed as the binding particle of the present embodiment capable of effectively adsorbing nucleic acids.

The above-mentioned electronic materials are significantly less expensive than the commercially available nucleic acid adsorption particles used in the Boom method. Therefore, by repurposing the silica particles, which are the electronic material, as the binding particle in the present embodiment, that is, by "using a silica particle, in which at least a portion of the surface is covered with silica and which has vicinal silanol groups on the surface, for nucleic acid adsorption by the Boom method", it is possible to produce nucleic acid-binding particles that are less expensive than conventional ones.

### <<Method for producing nucleic acid-binding particle>>

FIG. 2 is a flowchart illustrating a method for producing a nucleic acid-binding particle. The method for producing a nucleic acid-binding particle includes the following steps 1 to 4.

### (Step 1: Preparation of raw material particle)

First, a particle with a silica coating on its surface is prepared to serve as a raw material for a nucleic acid-binding particle (raw material particle).

The raw material particle only needs to have silica on at least a portion of its surface. That is, the raw material particle only needs to have silica exposed on at least a portion of its surface, and, for example, a portion of the particle surface may be coated with a resin. Preferably, the entire surface of the raw material particle is composed of silica. The interior of the raw material particle can have a structure common to the internal structure of the binding particle described in the above-mentioned section of [Particle structure].

### (Step 2: Adjustment of hydrophilicity of particle surface)

Next, the prepared raw material particle is subjected to hydrothermal treatment to control the hydrophilicity evaluation value S (evaluation value obtained from the following formula (B)) of the particle surface of the raw material particle to 0.35 or more. $S = {α}_{H 2 O} / {α}_{N 2}$ (B)
S: Hydrophilicity evaluation value of surface of particle
α_{H2O}: BET specific surface area of binding particle determined through water vapor adsorption measurement
α_{N2}: BET specific surface area of binding particle determined through nitrogen adsorption measurement.

In the present embodiment, the term "hydrothermal treatment" refers to a heating treatment of the raw material particle in the presence of high-temperature and highpressure water. The pressure during treatment can be set as appropriate as long as it can achieve the desired heating temperature. The hydrothermal treatment may also be replaced by superheated steam irradiation treatment.

Step 2 will be explained in detail.

The silanol groups in silica are reduced by dehydration and the formation of siloxane bonds through heat treatment in a dry environment. On the other hand, siloxane bonds are cleaved (depolymerized) by hydrothermal treatment, generating silanol groups.

Therefore, in the method for producing a nucleic acid-binding particle of the present embodiment, the amount of vicinal silanol groups on the particle surface can be increased by processing the raw material particle, whose surface is covered with silica, to generate and increase vicinal silanol groups, such as a hydrothermal treatment or a superheated steam treatment, thereby increasing the density of vicinal silanol groups. The amount of vicinal silanol groups on the particle surface can be adjusted by changing the conditions of the hydrothermal treatment. For example, if the amount of vicinal silanol groups after hydrothermal treatment is less than expected, it is possible to increase the amount of vicinal silanol groups by changing the conditions such as increasing the treatment time or raising the treatment temperature. In the present embodiment, the nucleic acid-binding particle can be produced by changing the treatment conditions to set the evaluation value to 0.35 or more.

The hydrothermal treatment is carried out, for example, by heating the raw material particle overnight in a temperature range of 100°C to 180°C. As an example, the hydrothermal treatment is carried out by heating the raw material particle overnight at 130°C.

In addition, when using a commercially available silica particle as a raw material to manufacture the binding particle, the silica particle may be heat-treated (calcined) in a dry environment prior to the hydrothermal treatment described above to remove surface silanol groups. In other words, Step 2 may be carried out in two stages: drying of the raw material and hydrothermal treatment of the raw material. This makes it possible to ignore the differences in the amount of surface silanol groups due to differences in the manufacturer or lot of the silica particle, and by performing a hydrothermal treatment under a constant treatment condition, it is possible to stably manufacture the silica particle (binding particle) with a desired density of vicinal silanol groups.

Heat treatment in a dry environment is carried out, for example, by calcining the raw material particle in a temperature range of 500°C to 1,000°C.

The conditions for the hydrothermal treatment can be set by conducting a preliminary experiment in advance. That is, the abundance ratio of vicinal silanol groups can be measured for the raw material particle subjected to the treatment in Step 2 above, and it is advisable to determine the conditions under which the abundance ratio of vicinal silanol groups is between 0.93 and 1.00. The hydrothermal treatment in Step 2 can then be performed under the conditions thus determined.

### (Step 3: Drying)

After the hydrothermal treatment, the supernatant is discarded, and the resulting particle is dried overnight. Thereby, a particle obtained by hydrothermally treating the raw material particle (nucleic acid-binding particle) is obtained. The drying process can be carried out by commonly known methods. For example, the drying process can be performed by heating, reduced pressure, blowing air, or a combination thereof. When drying is performed by heating, for example, it can be carried out at a temperature between room temperature and 250°C.

### (Step 4: Dispersion into the dispersion medium)

Next, the obtained nucleic acid-binding particle may be dispersed in sterile distilled water to prepare a dispersion with a predetermined particle concentration. The concentration of the dispersion may be 20 mg/mL or more and may also be 40 mg/mL or more. Alternatively, the concentration of the dispersion may be 200 mg/mL or lower and may also be 160 mg/mL or lower. The upper and lower limits of the dispersion concentration can be arbitrarily combined. For example, the concentration of the dispersion may be 20 mg/mL or more and 200 mg/mL or lower, or 40 mg/mL or more and 160 mg/mL or lower. In the examples described later, the concentration of the dispersion is 40 mg/mL. The manufactured nucleic acid-binding particle is ultimately supplied in the form of dispersion.

### <<Method for extracting nucleic acids>>

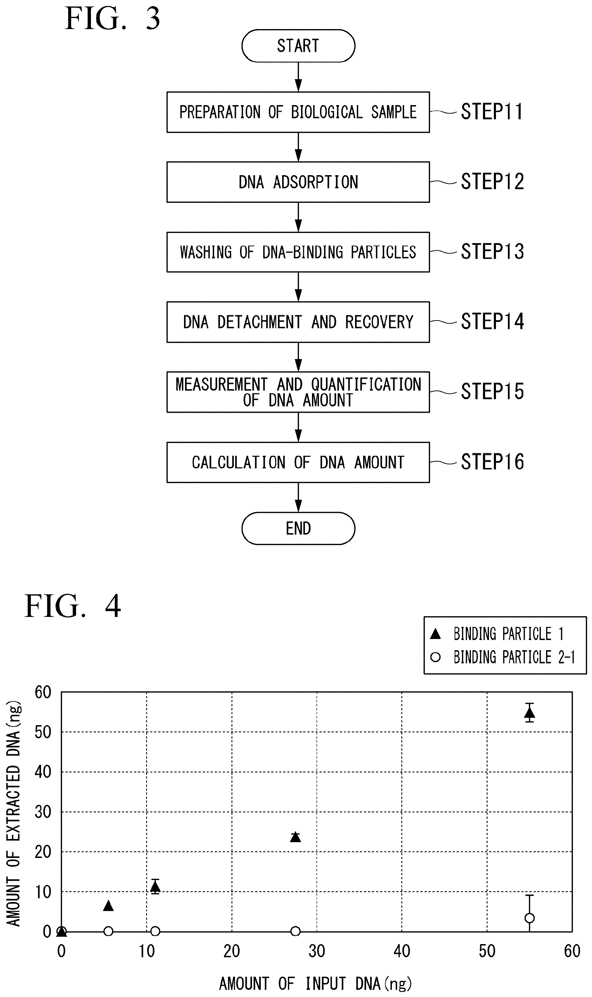

FIG. 3 is a flowchart illustrating a method for extracting nucleic acids using a nucleic acid-binding particle. The above-described method for extracting nucleic acids using a nucleic acid-binding particle includes the steps of: mixing a nucleic acid-binding particle with a biological sample containing nucleic acids to form a dispersion (Step 11); adsorbing nucleic acids onto the surface of the nucleic acid-binding particle (Step 12); washing the nucleic acid-binding particle separated from the dispersion with a washing solution (Step 13); and separating the nucleic acids from the washed nucleic acid-binding particle and recovering the nucleic acids (Step 14). Furthermore, the amount of recovered nucleic acids is measured and quantified (Step 15), and the amount of nucleic acids is calculated (Step 16).

Through studies by the inventors, it was clarified that, depending on the nucleic acid-binding particle used, if the washing solution used in the washing step contains alcohol, nucleic acids may detach from the nucleic acid-binding particle during the washing step, reducing the amount of nucleic acid recovered. The details are explained below.

### (Step 11: Preparation of biological sample)

First, a biological sample from which nucleic acids are extracted is prepared. For example, the biological sample is diluted with a known buffer to adjust its concentration. As a buffer, for example, 10 mmol/L tris(hydroxymethyl)aminomethane hydrochloride (pH 8.5) can be used. The binding particle is added to the aqueous solution of the obtained biological sample and shaken at room temperature for, for example, 10 minutes. The binding particle to be added may be the dispersion described above.

### (Step 12: DNA adsorption)

Next, a Lysis buffer containing 8 mol/L guanidine salt is added to the dispersion obtained in Step 11 to adsorb DNA onto the binding particle. The dispersion containing the binding particle is centrifuged, for example, at 700 rpm for 5 minutes to precipitate the binding particle. The separated Lysis buffer is discarded.

### (Step 13: Washing of DNA-binding particle)

Next, a buffer solution (washing solution) containing 10 mmol/L tris(hydroxymethyl)aminomethane hydrochloride and ethanol is added to the obtained binding particle. Then, the washing solution containing the dispersed binding particle is centrifuged at 700 rpm for 5 minutes to precipitate the binding particle. The separated washing solution is discarded to wash the binding particle.

If the process from adding the buffer solution to the binding particle to discarding the separated washing solution is considered a single washing operation, the washing operation of the binding particle may be performed multiple times. For example, the washing operation may be performed twice.

### (Step 14: DNA detachment and recovery)

The obtained binding particle is dispersed again with a buffer solution (extract) containing 10 mmol/L tris(hydroxymethyl)aminomethane hydrochloride (e.g., 60 µL), and the mixture is heated at 70°C for 10 minutes. The extract containing the dispersed binding particle is then centrifuged at 700 rpm for 10 minutes to precipitate the particle, and the supernatant (extract containing DNA, e.g., 60 µL) is recovered.

### (Step 15: Measurement and quantification of DNA amount)

A portion of the obtained supernatant (e.g., 2 µL) is used to quantify the DNA concentration of the supernatant using a DNA concentration analyzer and a detection reagent. As the DNA concentration analyzer, for example, a Qubit4 Fluorometer (manufactured by Thermo Fisher) can be used. Additionally, as the detection reagent, for example, a Qubit 1X dsDNA HS Assay Kit (manufactured by Thermo Fisher) can be used.

### (Step 16: Calculation of DNA amount)

The amount of extracted DNA is calculated by multiplying the measured concentration by the volume of the supernatant (60 µL).

A nucleic acid-binding particle with the above configuration can be used to efficiently recover nucleic acids from biological samples.

Furthermore, this method for producing a nucleic acid-binding particle allows for the easy production of the aforementioned nucleic acid-binding particle.

Although preferable embodiments of the present invention have been described above with reference to the attached drawings, the present invention is not limited to these examples. The shapes and combinations of the constituent members shown in the above examples are merely examples, and can be modified in various ways based on design, specifications, etc., without departing from the scope of the present invention.

### [Examples]

The present invention will be described below with reference to Examples, but the present invention is not limited to these Examples.

### [Hydrophilicity of particle surface]

In the present Examples, the nitrogen adsorption amount, water vapor adsorption amount, and BET specific surface area of the binding particle were determined according to the above-mentioned sections of [Particle surface state 1: Sphericity] and [Particle surface state 2: Hydrophilicity of particle surface], and the hydrophilicity evaluation value of the surface of the binding particle was calculated.

### [Particle sphericity]

In the present Examples, the sphericity of the binding particle was calculated according to the above-mentioned section of [Particle Surface State 1: Sphericity].

### [Raman spectroscopy]

In the present Examples, Raman spectroscopy of the binding particle was performed according to the above-mentioned section of (Raman spectroscopy).

### [Biological sample]

Biological sample 1: Dispersion containing λDNA (manufactured by Nippon Gene, concentration: 550 ng/µL)

Biological sample 2: A dispersion prepared by the following method.

### (Preparation method)

HeLa cells, which are a cell line established from human cervical cancer cells, were cultured and then counted using a Thoma hemocytometer. The cells were collected into tubes such that the number of cells in each sample was 1,000, 2,000, and 10,000. The cells in each tube were lysed using Proteinase K to prepare biological sample 2 containing DNA.

### (Common procedure: DNA extraction)

DNA extraction was performed based on the above-mentioned section of <<Method for extracting nucleic acids>>.

The biological sample was diluted with 10 mmol/L tris(hydroxymethyl)aminomethane hydrochloride (pH 8.5) and adjusted to each concentration used in the experiment. Into each tube containing the aqueous solution of the biological sample, 23.1 µL of a dispersion liquid of binding particle was added, followed by shaking at room temperature for 10 minutes (Step 11 in FIG. 3).

A Lysis buffer containing 8 mol/L guanidine salt was added to the resulting dispersion to adsorb DNA onto the binding particle. The dispersion containing the binding particle was centrifuged at 700 rpm for 5 minutes to precipitate the binding particle, and the separated Lysis buffer was discarded (Step 12 in FIG. 3).

A buffer solution (washing solution) containing 10 mmol/L tris(hydroxymethyl)aminomethane hydrochloride and ethanol was added to the binding particle. The washing solution containing the dispersed binding particle was then centrifuged at 700 rpm for 5 minutes to precipitate the binding particle, and the separated washing solution was discarded to wash the binding particle. This washing procedure was performed twice (Step 13 in FIG. 3).

The obtained binding particle was dispersed again by adding 60 µL of buffer solution (extract) containing 10 mmol/L tris(hydroxymethyl)aminomethane hydrochloride, and the mixture was heated at 70°C for 10 minutes. Subsequently, the extract containing the dispersed binding particle was centrifuged at 700 rpm for 10 minutes to precipitate the particle, and the supernatant (60 µL of extract containing DNA) was collected (Step 14 in FIG. 3).

A portion of the obtained supernatant (2 µL) was used to quantify the DNA concentration of the supernatant using a DNA concentration analyzer (Qubit4 Fluorometer, manufactured by Thermo Fisher) and a detection reagent (Qubit 1X dsDNA HS Assay Kit, manufactured by Thermo Fisher) (Step 15 in FIG. 3).

The amount of extracted DNA was calculated by multiplying the measured concentration by the volume of the supernatant (60 µL) (Step 16 in FIG. 3).

DNA extraction was performed on various binding particles based on the common procedure described above. The experimental results (Evaluation 1 to Evaluation 3) are described below.

### [Evaluation 1: Vicinal silanol groups]

In the following evaluation, commercially available silica particles which are used as electronic materials (gap materials) were used as the binding particles. Since gap materials have extremely uniform particle diameters, there is no significant difference between the particle diameter of each individual particle and the average particle diameter.

The particle diameter of binding particle 1 used in Evaluation 1, determined through image analysis, is as follows. Furthermore, the presence of vicinal silanol groups on the surface of binding particle 1 was confirmed by Raman spectroscopy, and the hydrophilicity of the particle surface was evaluated.

Binding particle 1: Particle diameter of 1 µm, hydrophilicity evaluation value of 0.51, sphericity of 0.89

Each binding particle was suspended in sterile distilled water to prepare a dispersion with a particle concentration of 40 mg/mL.

Evaluations were performed using the untreated binding particle 1 and binding particle 1 that was subjected to calcination treatment (heat treatment in a dry environment) shown in Table 1.

Using biological sample 1 and setting the amount of input DNA to 60 ng, DNA was adsorbed and extracted according to the common procedure described above. The evaluation results are shown in Table 1.

**[Table 1]**

| No. | Treatment | Calcination temperature | Hydrophilicity evaluation value | Amount of input DNA (ng) | Amount of extracted DNA (ng) |
|---|---|---|---|---|---|
| 1-1 | Untreated | - | 0.51 | 60.0 | 45.78 |
| 1-2 | Calcination | 250°C | 0.45 | | 43.5 |
| 1-3 | | 500°C | 0.35 | | N.D. |
| N.D.: Not detected | | | | | |

The evaluation results showed that the sample treated at 250°C exhibited the same level of DNA adsorption capacity as the untreated sample, while the samples treated at 500°C, 750°C, and 1,000°C all lost their capability to adsorb DNA. It is considered that the calcination treatment reduced the number of vicinal silanol groups on the surface, resulting in the loss of adsorption capacity.

Furthermore, as will be described later, DNA can be recovered from the binding particle No. 1-3 by adjusting the composition of the washing solution.

The binding particle No. 1-3 in Table 1 was subjected to hydrothermal treatment at 130°C overnight. Using biological sample 1 and setting the input amount of DNA to 51 ng, DNA was adsorbed and extracted according to the common procedure described above, using the untreated binding particle 1 (No. 1-1 in Table 1) and the hydrothermally treated binding particle. The evaluation results are shown in Table 2. The hydrophilicity evaluation value for the hydrothermally treated binding particle (No. 1-4 in Table 2) was measured to be 0.63.

**[Table 2]**

| No. | Treatment | Amount of input DNA (ng) | Amount of extracted DNA (ng) |
|---|---|---|---|
| 1-1 | Untreated | 51.0 | 49.9 |
| 1-4 | Hydrothermal treatment | | 51.1 |

The evaluation results showed that the particle subjected to hydrothermal treatment after calcination regained DNA adsorption capacity, similar to the untreated particle. This strongly suggests that hydrothermal treatment of the particle leads to the formation of vicinal silanol groups on the particle, thereby acquiring DNA adsorption capacity.

### [Evaluation 2: Influence of surface shape of binding particle]

In Evaluation 2, the influence exerted by the surface shape was examined using the aforementioned binding particle 1 and binding particles with different sphericities. First, the influence of the shape was examined using binding particle 1, and binding particle 2-1 which is a porous particle. The particle diameter of binding particle 2-1, determined through image analysis, is as follows. The presence of vicinal silanol groups on the surface of binding particle 2-1 was confirmed by Raman spectroscopy, and the hydrophilicity of the particle surface was evaluated.

Binding particle 2-1: Particle diameter of 1 µm, hydrophilicity evaluation value of 0.38, sphericity of 0.01

Using biological sample 1 and setting the amount of input DNA to 55 ng, DNA was adsorbed and extracted according to the common procedure described above.

The evaluation results are shown in FIG. 4 and Table 3 below. FIG. 4 is a graph showing the evaluation results.

**[Table 3]**

| | Particle diameter (µm) | Sphericity | DNA recovery rate (%) |
|---|---|---|---|
| Binding particle 1 | 1 | 0.89 | 94.7 |
| Binding particle 2-1 | 1 | 0.01 | 5.7 |

As shown in FIG. 4, it was found that binding particle 1, which has a sphericity close to 1 and a relatively smooth surface, was able to recover more DNA than binding particle 2-1, which has a lower sphericity and a rougher surface. From the above results, it was found that for the nucleic acid-binding particle of the present invention, a particle with a smooth surface is advantageous, because a surface structure that increases the surface area, such as a porous structure acts disadvantageously on the DNA yield.

Furthermore, the influence of the shape was investigated using binding particle 2-2, which is formed from the same material as binding particle 1 but has a slightly lower sphericity than binding particle 1. The particle diameter of binding particle 2-2, determined through image analysis, is as follows. The presence of vicinal silanol groups on the surface of binding particle 2-2 was confirmed by Raman spectroscopy, and the hydrophilicity of the particle surface was evaluated.

Binding particle 2-2: Particle diameter of 1 µm, hydrophilicity evaluation value of 44.25, sphericity of 0.86

Furthermore, binding particle 2-2 showed a high hydrophilicity evaluation value of 44.25, which is considered to be due to the shape of the particle surface of binding particle 2-2. Because binding particle 2-2 had a low sphericity, it was considered to have gentle irregularities or undulations on its particle surface compared to other binding particles. It can be presumed that, due to such a surface shape, binding particle 2-2 showed a tendency for water to be easily adsorbed and condensed on the hydroxyl groups. For this reason, it can be presumed that when measuring the hydrophilicity of binding particle 2-2, the BET specific surface area (α_{H2O}) of the binding particle obtained by water vapor adsorption measurement tended to show a large value, resulting in a high evaluation value.

Using biological sample 2 containing 10,000 cells, DNA was adsorbed and extracted according to the common procedure described above. The evaluation results are shown in Table 4 below.

**[Table 4]**

| | Sphericity | Number of input cells | Amount of extracted DNA (ng) |
|---|---|---|---|
| Binding particle 1 | 0.89 | 10,000 | 119.8 |
| Binding particle 2-2 | 0.86 | | 48.8 |

As shown in Table 4, compared to binding particle 1, which had a sphericity of 0.89, the amount of extracted DNA was significantly reduced with binding particle 2-2, which had a sphericity of 0.86, but it was found that DNA could still be recovered.

In the above Examples, although the effect was confirmed by adsorbing DNA onto the binding particles, it is assumed that similar behavior would occur with RNA due to the similarity in structure.

### [Evaluation 3: Hydrophilicity of particle surface]

It was found that binding particle 1 retained the capability to adsorb DNA when untreated and when subjected to the calcination treatment at 250°C, but lost its DNA-adsorbing capability at 500°C. On the other hand, as for the influence exerted on the DNA yield, it is necessary to consider not only the adsorption process but also the washing process (Step 13 in FIG. 3) which is usually performed for the purpose of increasing the purity of DNA. Therefore, the following experiment was conducted. Note that the sphericity of binding particles No. 3-1 to No. 3-3 used in Evaluation 3 were not affected by the calcination treatment and were 0.89, which is the same as that of binding particle 1.

Using binding particle 1 or binding particle 1 calcined at 500°C, DNA was adsorbed and extracted according to the common procedure shown in FIG. 3. At that time, either of the following washing solutions 1 and 2 was used as the washing solution in Step 13. Washing solution 2 is the washing solution used in the common procedure.

Washing solution 1: a 10 mmol/L tris(hydroxymethyl)aminomethane hydrochloride aqueous solution not containing 70% ethanol

Washing solution 2: a 10 mmol/L tris(hydroxymethyl)aminomethane hydrochloride aqueous solution containing 70% ethanol

The evaluation results are shown in Table 5.

**[Table 5]**

| No. | Treatment | Hydrophilicity evaluation value | Washing solution (Ethanol presence/absence) | Amount of input DNA (ng) | Amount of extracted DNA (ng) |
|---|---|---|---|---|---|
| 3-1 | Untreated | 0.51 | Washing solution 1 (absence) | | 28.8 |
| 3-2 | Calcination at 500°C | 0.35 | Washing solution 1 (absence) | 55.0 | 22.0 |
| 3-3 | Calcination at 500°C | 0.35 | Washing solution 2 (presence) | | 1.4 |

From the results for Nos. 3-1 and 3-2, it was found that although the particles calcined at 500°C can adsorb DNA, the amounts of DNA that can be adsorbed were less than that of the untreated particle (No. 3-1 in Table 5). The particles calcined at 500°C had lower hydrophilicity values than that of the untreated particle, indicating a decrease in silanol groups on the particle surface.

On the surface of the binding particle, vicinal silanol groups and water molecules form hydrogen bonds, and it is considered that DNA is bound to the vicinal silanol groups via the water molecules. When the washing solution does not contain ethanol, electrostatic repulsion is shielded by the salts in the washing solution, allowing DNA and the binding particle to bind via the water molecules. From the comparison results of No. 3-1 and No. 3-2, which did not contain ethanol in the washing solution, it was found that the hydrophilicity evaluation value changed depending on whether or not calcination was performed, and that the amount of extracted DNA also decreased in accordance with the decrease in the hydrophilicity evaluation value. Therefore, it is considered that calcination reduces the amount of vicinal silanol groups.

Furthermore, from the results of No. 3-2 and 3, it was found that when the particles calcined at 500°C were used, washing them with a washing solution containing ethanol, similar to Evaluation 1 above, significantly reduced the amount of DNA that could be extracted. This phenomenon can be explained as follows.

First, if the washing solution contains ethanol, it is considered that some of the vicinal silanol groups on the surface of the binding particle will be esterified, preventing them from forming hydrogen bonds with water molecules. As a result, the amount of vicinal silanol groups that can bind to DNA via water molecules is reduced.

Furthermore, on the binding particle to which DNA is adsorbed, the DNA is considered to be in an equilibrium state of repeating adsorption to and desorption from the vicinal silanol groups. Hydrogen bonds are considered to be formed with water on the polar groups of the DNA that is not adsorbed onto the vicinal silanol groups, thereby stabilizing the DNA in the supernatant in which it is dispersed.

Here, if the washing solution contains ethanol, the hydrogen-bonded water in the DNA in the supernatant is dehydrated by the ethanol, making it easier for the polar groups of the DNA to bind to each other within or between DNA molecules. In DNA that has bound in this way, the sites where vicinal silanol groups would bind (polar groups) are consumed by binding within or between DNA molecules, making it less likely for them to bind to vicinal silanol groups. In other words, if the washing solution contains ethanol, the amount of DNA that readily binds to vicinal silanol groups is thought to decrease.

When the washing solution contains ethanol, it is considered that (i) a reduction in the amount of vicinal silanol groups that can bind to DNA and (ii) a reduction in the amount of DNA that readily binds to vicinal silanol groups occur in combination, making it difficult for the binding particle to retain DNA during washing. As a result, when the washing solution contains ethanol, it is considered that DNA is washed away during washing, and the amount of DNA extracted is reduced.

When the amount of vicinal silanol groups in a binding particle is small, the total amount of vicinal silanol groups that have not been esterified and are capable of adsorbing DNA is also small. Therefore, DNA adsorption by vicinal silanol groups is less likely to occur, and even if DNA is adsorbed by vicinal silanol groups, the adsorption force is weaker compared to a binding particle with a relatively large total amount of vicinal silanol groups. As a result, a binding particle with few vicinal silanol groups is more strongly affected by ethanol in the washing water than a binding particle with many vicinal silanol groups, making DNA extraction more difficult.

As described above, the ethanol contained in the washing solution is considered to affect the amount of DNA extracted by a binding particle. As shown in Table 5, when the binding particle was washed with washing solution 2 containing ethanol (No. 3-3), the DNA-adsorbing force of the binding particle was weaker compared to when the binding particle was washed with washing solution 1 without ethanol (No. 3-2), which is considered to have resulted in a smaller amount of extracted DNA.

On the other hand, it was confirmed that if the hydrophilicity evaluation value is 0.35, DNA can be extracted by employing an alcohol-free aqueous solution as the washing solution for the binding particle.

### [Evaluation 4: Examination of particle diameter of binding particle]

In this evaluation, the effect of particle diameter on the binding particle was investigated using binding particles 3 and 4, which were formed from the same material as binding particle 1 but differed only in particle diameter. The particle diameters of binding particles 3 and 4, determined through image analysis, are as follows. The presence of vicinal silanol groups on the surfaces of binding particles 3 and 4 was confirmed by Raman spectroscopy, and the hydrophilicity of the particle surface was evaluated.

Binding particle 3: Particle diameter of 0.16 µm, hydrophilicity evaluation value of 0.41, sphericity of 0.97

Binding particle 4: Particle diameter of 50 µm, hydrophilicity evaluation value of 8.26, sphericity of 1

Since binding particles 3 and 4 were made from the same forming material as binding particle 1, differing only in particle diameter, it is assumed that the distribution of vicinal silanol groups on their surfaces is the same as that of binding particle 1. However, the hydrophilicity evaluation value of binding particle 4 differs significantly from that of binding particles 1 and 3, despite being made from the same forming material. In response to this, the inventors found that the hydrophilicity evaluation value tends to increase as the particle diameter of the binding particle increases.

Since BET specific surface area is used in calculating the hydrophilicity evaluation value, it is considered to be strongly influenced by the sample amount (total surface area) of the binding particle used to measure the specific surface area. Although the calculated hydrophilicity evaluation value of binding particle 4 differs significantly from that of binding particles 1 and 3 as described above, based on the above findings, the actual hydrophilicity of binding particle 4 is assumed to be the same as that of binding particles 1 and 3, and the following evaluation was performed.

### (Evaluation 4-1)

The effect of particle diameter was investigated using binding particle 1 and binding particle 3. For binding particle 3, the concentration of the dispersion was set to 6.4 mg/mL, and the total surface area of the particle in the dispersion was prepared to be the same as that of the dispersion of binding particle 1.

Using biological sample 1, DNA was adsorbed and extracted according to the common procedure described above, with the input amount being 5.5 ng or 11.0 ng.

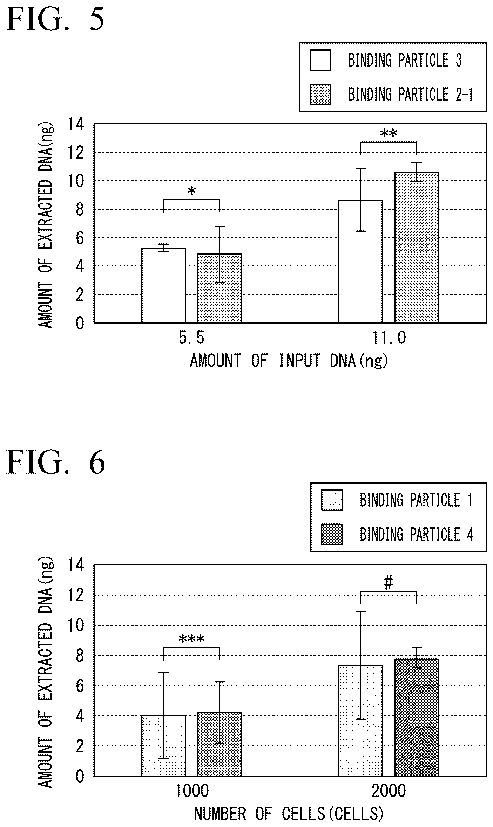

FIG. 5 is a graph showing the evaluation results. As shown in FIG. 5, no difference was observed between binding particle 3, which has a particle diameter of 0.16 µm, and binding particle 1, which has a particle diameter of 1 µm (for both * and ** in FIG. 5, p > 0.05, indicating no significant difference).

### (Evaluation 4-2)

The effect of particle diameter on DNA extraction was investigated using binding particle 1 and binding particle 4. For binding particle 4, the concentration of the dispersion was set to 2,000 mg/mL, and the total surface area of the particle in the dispersion was prepared to be the same as that of the dispersion of binding particle 1.

In this evaluation, two biological samples containing 1,000 or 2,000 cells were used, and DNA was adsorbed and extracted according to the common procedure described above.

FIG. 6 is a graph showing the evaluation results. As shown in FIG. 6, no difference was observed between binding particle 1, which has a particle diameter of 1 µm, and binding particle 4, which has a particle diameter of 50 µm (for both *** and # in FIG. 6, p > 0.05, indicating no significant difference).

Furthermore, when evaluating the hydrophilicity of various binding particles, within the examined range, the highest value was "125.54" for a binding particle formed from the same material as binding particle 1, with a sphericity of 0.86 or more, and a particle diameter of 10 µm determined through image analysis. It was also confirmed that DNA could be adsorbed and extracted using this binding particle according to the common procedure described above.

### [Evaluation 5: Influence of impurities in biological samples]

As shown in Table 6 below, a sample containing λDNA and the proteolytic enzyme Proteinase K, and a sample without these components were prepared.

If the sample contained the proteolytic enzyme Proteinase K, the concentration of Proteinase K was set to 10 mg/mL (final concentration).

Using the prepared samples, DNA was adsorbed and extracted according to the common procedure described above.

The results are shown in Table 6. In the table, (+) means that the target substance is present in the sample, and (-) means that the target substance is not present in the sample.

**[Table 6]**

| λDNA | Proteinase K | Amount of input DNA (ng) | Amount of extracted DNA (ng) |
|---|---|---|---|
| (+) | (+) | | 45.2 |
| (+) | (-) | 50.0 | 44.76 |
| (-) | (+) | | N.D. |
| (-) | (-) | | N.D. |
| N.D.: Not detected | | | |

The evaluation confirmed that Proteinase K, a proteolytic enzyme contained in the sample, does not affect the adsorption of DNA to the binding particles.

From the above results, it was confirmed that the present invention is useful.

### REFERENCE SIGNS LIST

A ... Surface silanol group, A1 ... Vicinal silanol group, A2 ... Isolated silanol group, B ... Internal silanol group

## Claims

1. A nucleic acid-binding particle, comprising at least a portion of a surface covered with silica, wherein
a sphericity determined from the following formula (A) is 0.86 or more, and the surface has vicinal silanol groups: 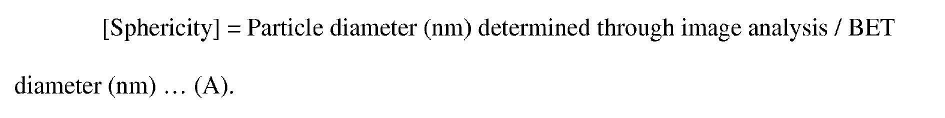 [Sphericity] = Particle diameter (nm) determined through image analysis / BET diameter (nm) (A).

2. The nucleic acid-binding particle according to claim 1, wherein
a hydrophilicity evaluation value of the surface determined from the following formula (B) is 0.35 or more: $S = {α}_{H 2 O} / {α}_{N 2}$ (B)
S: Hydrophilicity evaluation value of surface of particle
α_{H2O}: BET specific surface area of binding particle determined through water vapor adsorption measurement
α_{N2}: BET specific surface area of binding particle determined through nitrogen adsorption measurement.

3. The nucleic acid-binding particle according to claim 2, wherein
an abundance ratio of the vicinal silanol groups on the surface determined from the following formula (C) is 0.93 or more and 1.00 or less: $\left[Abundance ratio of vicinal silanol groups\right] = Rv / Rt$ (C)
Rv: Peak area of vicinal silanol groups in Raman spectrum
Rt: Total peak area of vicinal silanol groups and isolated silanol groups in Raman spectrum.

4. The nucleic acid-binding particle according to any one of claims 1 to 3, having an average particle diameter of 0.1 µm or more and 1,000 µm or less.

5. The nucleic acid-binding particle according to claim 4, wherein the average particle diameter is 0.1 µm or more and 200 µm or less.

6. The nucleic acid-binding particle according to any one of claims 1 to 3, comprising a magnetic material therein.

7. A method for producing a nucleic acid-binding particle, comprising:
controlling a hydrophilicity evaluation value of a surface determined from the following formula (B) to 0.35 or more by subjecting a particle whose surface is covered with silica to a treatment for increasing vicinal silanol groups: $S = {α}_{H 2 O} / {α}_{N 2}$ (B)
S: Hydrophilicity evaluation value of surface of particle
α_{H2O}: BET specific surface area of binding particle determined through water vapor adsorption measurement
α_{N2}: BET specific surface area of binding particle determined through nitrogen adsorption measurement.

8. The method for producing a nucleic acid-binding particle according to claim 7, wherein the treatment for increasing vicinal silanol groups is a hydrothermal treatment of the particle.

9. The method for producing a nucleic acid-binding particle according to claim 8, wherein
the nucleic acid-binding particle has an abundance ratio of the vicinal silanol groups on the surface determined from the following formula (C) of 0.93 or more and 1.00 or less: $\left[Abundance ratio of vicinal silanol groups\right] = Rv / Rt$ (C)
Rv: Peak area of vicinal silanol groups in Raman spectrum
Rt: Total peak area of vicinal silanol groups and isolated silanol groups in Raman spectrum.

10. The method for producing a nucleic acid-binding particle according to any one of claims 7 to 9, wherein the particle is heat-treated in a dry environment prior to the hydrothermal treatment of the particle.

11. The method for producing a nucleic acid-binding particle according to any one of claims 7 to 9, wherein
a sphericity of the particle determined from the following formula (A) is 0.86 or more: 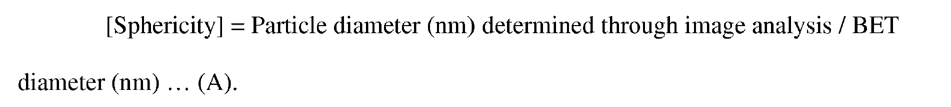 [Sphericity] = Particle diameter (nm) determined through image analysis / BET diameter (nm) (A).
